(19) Europäisches Patentamt / European Patent Office / Office européen des brevets

(11) **EP 3 335 001 B1**

(12) **EUROPÄISCHE PATENTSCHRIFT**

(45) Veröffentlichungstag und Bekanntmachung des Hinweises auf die Patenterteilung:
**12.06.2019  Patentblatt 2019/24**

(51) Int Cl.:
*G01B 9/02* *(2006.01)*          *A61B 3/10* *(2006.01)*
*A61B 5/00* *(2006.01)*          *G01N 21/47* *(2006.01)*
*G03H 1/04* *(2006.01)*          *G03H 1/06* *(2006.01)*

(21) Anmeldenummer: **16756634.8**

(22) Anmeldetag: **09.08.2016**

(86) Internationale Anmeldenummer:
**PCT/EP2016/068975**

(87) Internationale Veröffentlichungsnummer:
**WO 2017/029160 (23.02.2017 Gazette 2017/08)**

(54) **VERFAHREN UND VORRICHTUNG ZUM ABLICHTEN WENIGSTENS EINER SCHNITTFLÄCHE IM INNERN EINES LICHT STREUENDEN OBJEKTS**

METHOD AND DEVICE FOR CAPTURING AT LEAST ONE SECTIONAL IMAGE OF THE INTERIOR OF A LIGHT-SCATTERING OBJECT

PROCÉDÉ ET DISPOSITIF POUR PHOTOGRAPHIER AU MOINS UNE SURFACE DE COUPE À L'INTÉRIEUR D'UN OBJET DIFFUSANT LA LUMIÈRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.08.2015  DE 102015113465**

(43) Veröffentlichungstag der Anmeldung:
**20.06.2018  Patentblatt 2018/25**

(73) Patentinhaber:
• **Thorlabs GmbH**
  **85221 Dachau (DE)**
• **Medizinisches Laserzentrum Lübeck GmbH**
  **23562 Lübeck (DE)**

(72) Erfinder:
• **KOCH, Peter**
  **23558 Lübeck (DE)**
• **FRANKE, Gesa**
  **60327 Frankfurt (DE)**
• **SPAHR, Hendrik**
  **23562 Lübeck (DE)**
• **SUDKAMP, Helge**
  **23552 Lübeck (DE)**
• **HÜTTMANN, Gereon**
  **23564 Lübeck (DE)**
• **HILLMANN, Dierck**
  **23552 Lübeck (DE)**
• **BIRNGRUBER, Reginald**
  **23564 Lübeck (DE)**

(74) Vertreter: **Hermann, Felix**
  **Boehmert & Boehmert**
  **Anwaltspartnerschaft mbB**
  **Pettenkoferstrasse 22**
  **80336 München (DE)**

(56) Entgegenhaltungen:
**WO-A2-2009/111609      DE-A1-102006 015 387**

• **KWAN JEONG ET AL: "Fourier-domain digital holographic optical coherence imaging of living tissue", APPLIED OPTICS, OPTICAL SOCIETY OF AMERICA, WASHINGTON, DC; US, Bd. 46, Nr. 22, 6. Juli 2007 (2007-07-06), Seiten 4999-5008, XP007903279, ISSN: 0003-6935, DOI: 10.1364/AO.46.004999**
• **DUBOIS A ET AL: "Ultrahigh-resolution full-field optical coherence tomography", APPLIED OPTICS, OPTICAL SOCIETY OF AMERICA, WASHINGTON, DC; US, Bd. 43, Nr. 14, 10. Mai 2004 (2004-05-10), Seiten 2874-2883, XP002443955, ISSN: 0003-6935, DOI: 10.1364/AO.43.002874**

EP 3 335 001 B1

**Beschreibung**

**TECHNISCHES GEBIET DER ERFINDUNG**

Die Erfindung betrifft ein Verfahren und eine Vorrichtung zum Ablichten einer oder mehrerer Schnittflächen innerhalb eines Licht streuenden Objekts. Dabei kann kurzkohärentes Licht in einen Proben- und einen Referenzstrahl aufgeteilt, das Objekt mit dem Probenstrahl beleuchtet und das vom Objekt gestreute Licht auf einem elektronischen Flächendetektor, i. F. Kamera, mit dem Referenzstrahl zur Interferenz gebracht werden. Die Erfindung betrifft insofern ein Verfahren der kurzkohärenten bzw. Weißlicht-Interferometrie und eine dieses Verfahren ausführende Vorrichtung.

**TECHNISCHER HINTERGRUND DER ERFINDUNG**

Die vorliegende Beschreibung versteht unter "Ablichten" das Erfassen von Licht, das wenigstens teilweise von einem streuenden Objekt gestreut wird und unter physikalischem Manipulieren des Lichts zu einer Kamera gelangt. Das Manipulieren umfasst dabei sowohl das optische Abbilden des Objekts als auch das Überlagern von Probenlicht und Referenzlicht in einem Interferometer.

Die optische Kohärenztomographie ("Optical Coherence Tomography", OCT) ist heute eines der wichtigsten diagnostischen Verfahren in der Ophthalmologie. Seit der Markteinführung 1996 durch Humphrey Instruments und Zeiss haben sich Auflösung und Messgeschwindigkeit deutlich erhöht. Gerätegröße, Kosten und Bedienkonzept sind aber im Wesentlichen unverändert geblieben. OCT-Systeme sind bis heute üblich nur in Krankenhäusern und gut ausgestatteten Arztpraxen vorzufinden.

Für viele ophthalmologische Krankheitsbilder ist allerdings eine engmaschige Überwachung des Patienten und etwaiger Therapieeffekte wünschenswert. Beispiele dafür sind insbesondere die medikamentöse Injektionstherapie der feuchten Form der altersbedingten Makuladegeneration (AMD), aber auch andere Erkrankungen wie diabetische Makulaödeme oder retinale Venenverschlüsse. Die Verlaufskontrolle einer Therapie sollte mit einer häufigen, wenn nicht sogar täglichen, tiefenaufgelösten Beobachtung des Augenhintergrunds verbunden sein, wie sie nur ein OCT-Gerät gestattet. Wegen der großen Patientenzahlen (ca. 1.8 Mio. AMD-Patienten in Deutschland) ist es in der Durchführung problematisch und auch kostenintensiv, wenn der Patient häufig Orte aufsuchen muss, wo OCT-Geräte vorhanden sind.

Es wäre daher wünschenswert, ein einfaches, leicht bedienbares und vor allem auch preiswertes OCT-Gerät zur Verfügung zu haben, das der Patient eigenhändig zu Hause benutzen kann.

Zum Erreichen dieses Ziels wäre es wünschenswert, die Kosten der OCT-Geräte siginifikant, z.B. um den Faktor 5-10, zu senken. Ferner wäre es für das Erreichen dieses Ziels vorteilhaft, wenn die Benutzung der OCT-Geräte durch ungeschultes Personal möglich ist. Insbesondere wäre es vorteilhaft, die Bewegungsartefakte zu kompensieren, die bei einer handgehaltenen Messung durch einen älteren Patienten entstehen.

Ein "Homecare"-OCT-Gerät wird üblicherweise eine Sequenz elektronischer Bilder von Schnittflächen der Retina erfassen und abspeichern. Aus diesen kann das Gewebevolumen in Form eines Tomogramms rekonstruiert werden. Dadurch wird eine für den Arzt interpretierbare Darstellung der Strukturen der Retina ermöglicht.

Die Zeitspanne, in der die gesamte Sequenz erfasst und gespeichert wird, soll im Folgenden als Messdauer bezeichnet werden. Die Messdauer sollte höchstens einige Sekunden betragen. Jedes einzelne Bild der Sequenz wird dabei in einem sehr viel kürzeren Zeitintervall erfasst, das nachfolgend als Belichtungszeit bezeichnet wird. Die Belichtungszeit entspricht der Integrationszeit für die Intensitäten auf dem Bildsensor der Kamera. Davon zu unterscheiden ist die Auslesezeit der Daten aus der Kamera. Sie entspricht dem Zeitintervall, das nötig ist, um die in den Pixeln akkumulierten elektrischen Ladungen zunächst in Spannungen und dann in digitale Informationen umzuwandeln. Die Auslesezeit begrenzt üblich die Bildrate einer Kamera.

In der OCT sind zwei unterschiedliche Bewegungsartefakte zu unterscheiden. Beide entstehen durch Bewegungen des Objekts relativ zur Messvorrichtung. Bewegungen in lateraler oder axialer Richtung auf einer Zeitskala in der Größenordnung der Messdauer führen dazu, dass sich die geometrische Zuordnung der gemessenen Strukturen im Verlauf der Messung ändert. Es kommt zu einer geometrischen Verzerrung des Tomogramms. Die wahrscheinlichste Ursache solcher Verzerrungsartefakte ist der Patient, dem es womöglich nicht gelingt, das OCT-Gerät während der Messdauer ruhig zu halten und mit dem Auge einen bestimmten Punkt zu fokussieren.

Demgegenüber kann es weiterhin zu einer axialen Bewegung der Retina innerhalb der Belichtungszeit kommen, die dann zu einer Phasenverschiebung des OCT Signals führt. Insbesondere besteht die Möglichkeit der völligen Auslöschung des Signals, wenn sich das Objekt während der Belichtungszeit um eine halbe Wellenlänge in axialer Richtung verschiebt. Für die Anwendung der OCT in der Augenheilkunde müssen Belichtungszeiten kleiner als 200 Mikrosekunden erreicht werden, um dieses Artefakt weitgehend zu vermeiden.

Zudem lässt sich bei der Untersuchung der Retina mit einem handgehaltenen OCT-Gerät der Abstand vom Gerät zum Auge nicht gut kontrollieren. Ein handgehaltenes OCT-Gerät sollte daher einen Messbereich von mindestens einem Zentimeter aufweisen, um die Retina bei beliebigen Patienten ohne weitere Vorbereitung sicher zu erfassen.

Seit einigen Jahren werden Holographie-Verfahren entwickelt, um die Streuerverteilung in diffus streuenden Objekten zu ermitteln. Man verwendet dabei eine durchstimmbare Laserlichtquelle, also räumlich und zeitlich kohärentes Licht mit variabler Wellenlänge, teilt das Licht in Proben- und Referenzstrahl auf und beleuchtet das Objekt mit dem Probenstrahl flächig. Das vom Objekt wiederkehrende Licht wird entweder gar nicht oder ins Unendliche abgebildet. Ein im Probenstrahl angeordneter Flächendetektor misst somit im Fernfeld des Objektwellenfeldes. Auf dem Flächendetektor wird dieses Wellenfeld mit dem des Referenzstrahls überlagert, wobei die gesamte Objekttiefe aufgrund der großen Kohärenzlänge des Laserlichts zu Interferenzen beiträgt. Das Messergebnis ist ein komplex strukturiertes Intensitätsmuster auf dem Detektor, das als Hologramm gespeichert wird. Man nimmt eine Mehrzahl von Hologrammen bei verschiedenen Wellenlängen auf. Das Wellenfeld des Referenzstrahls ist jeweils bekannt und wird in der späteren Auswertung im Computer dazu benutzt, aus den Hologrammen das Wellenfeld des Probenstrahls auf dem Detektor zu rekonstruieren. Dieses Wellenfeld kann numerisch in beliebige Tiefen des Objekts propagiert werden, um die dreidimensionale Lage von Streuzentren zu bestimmen.

Weitere Einzelheiten zu Verfahren, die sich der Methoden der Digitalen Holografie (DH) bedienen, sind etwa den Druckschriften US 2008/137933 A1 und US 2014/092392 A1 zu entnehmen. Bei diesen Verfahren ist die erreichbare Messtiefe durch die Linienbreite der Lichtquelle heute auf wenige Millimeter begrenzt. Eine Verschiebung des Objekts innerhalb der Belichtungszeit der Kamera führt direkt zu einer Reduzierung des Kontrastes des Hologramms. Sie ändert die Frequenz oder die Phasenlage der gemessenen Sinusmodulation. Damit ist dieses Verfahren anfällig gegen Bewegungsartefakte. Abgesehen davon, dass eine durchstimmbare Laserlichtquelle heute noch keine billige Komponente ist, könnten zur Bewegungskompensation noch weitere Komponenten zur Bewegungsmessung, etwa Beschleunigungssensoren, erforderlich sein.

Aus der Druckschrift von Massatsch et al., ("Time-domain optical coherence tomography with digital holographic microscopy", Applied Optics, Vol. 44, No. 10, 1 April 2005) geht ein Holografie-Verfahren hervor, bei dem ein Kohärenzfenster ("low coherence gating") eingeführt wird, um Interferenzen auf der Kamera von vornherein auf Strahlung aus jenen Tiefenschichten zu begrenzen, deren optische Weglänge bis zur Kamera mit der Weglänge des Referenzlichts innerhalb der Kohärenzlänge übereinstimmt. Für einen Tiefenscan des streuenden Objekts wird der Referenzspiegel verschoben, wie man dies aus der Time-Domain (TD)-OCT kennt. Man gewinnt hierbei Hologramme, die verschiedenen Tiefenebenen des Objekts zugeordnet werden können. Die Hologramme gestatten die holografische Rekonstruktion der Strukturinformation ganzer Objektebenen, Als Lichtquelle wird dort ein Pulslaser mit 80 Femtosekunden Pulsdauer verwendet.

Bei den vorgenannten holografischen Verfahren sind die erzielten Bilder nicht unmittelbar interpretierbar, sondern bedürfen der - im Allgemeinen rechenintensiven - numerischen Auswertung, um Strukturen in Ortsraumkoordinaten, d.h. ein verständliches Modell des Objekts, zu ermitteln.

Es gibt interessanterweise eine seit Langem bekannte Vorrichtung zur Vermessung von Oberflächenstrukturen von diffus streuenden Objekten, deren Aufbau dem von Massatsch et al. sehr ähnlich ist. Diese ist in der DE 4 108 944 A1 offenbart. Anders als in der Holografie wird hier die Oberfläche auf die Kamera abgebildet, so dass die Ortszuweisung der Strukturen durch das erhaltene Auflichtbild unproblematisch ist. Das Oberflächenprofil des Objekts kann mittels eines verschiebbaren Referenzspiegels abgetastet werden, wobei man faktisch den Abstand der Kamera zu den Probenlicht streuenden Oberflächenpunkten, die zur Interferenz mit dem Referenzlicht auf der Kamera beitragen, variiert.

Ein Nachteil der Vorrichtung der DE 4 108 944 A1 liegt im Auftreten von Speckle im Interferenzbild, deren Phasenlage a priori unbekannt ist. Die zufälligen Intensitäten und Phasen der Speckle lassen Rückschlüsse auf die Tiefe der streuenden Objektoberfläche erst zu, wenn diese Phasenlage durch zusätzliche Bildaufnahmen aufgeklärt worden ist. Hierzu ist der Referenzspiegel bei jeder vorbestimmten Position wenigstens einmal so zu verschieben, dass sich die Phasenlage aller Speckle ändert. Man benötigt insofern neben dem Referenzspiegel mit einem Antrieb ausgebildet zum schnellen Verfahren des Spiegels um die gewünschte Messtiefe im Objekt, in der Größenordnung Zentimeter, auch noch einen Phasenmodulator oder einen zweiten Antrieb mit einer Stellgenauigkeit der Größenordnung 10 Nanometer. Dies macht die Vorrichtung nicht nur teuer, sondern wohl auch empfindlich gegenüber Erschütterungen und Bewegungen aller Art. Sie erscheint ungeeignet für ein handgehaltenes Messgerät.

Aus der Druckschrift US 2010/020328 A1 geht ein Verfahren zur Detektion von OCT-Signalen mittels Zeilensensoren hervor, das eine Lösung zur Vermeidung von Unterabtastung auch für große Messtiefenintervalle auf dem Detektor vorschlägt. Dort wird insbesondere aufgezeigt, dass es mittels Beugung an einem Gitter möglich ist, die Ausbreitungsrichtung - und damit die Phasenfront - eines Lichtstrahls um einen Winkel zu ändern ohne gleichzeitig die Kohärenzebene - auch oft als Pulsfront bezeichnet - mit zu verkippen. Der hinter dem Gitter in Richtung auf ein Beugungsnebenmaximum propagierende Lichtstrahl kann entlang seines Strahlquerschnitts eine Weglängenverteilung aufweisen, die dafür sorgt, dass Licht vom linken und rechten Strahlrand bereits nicht mehr miteinander interferiert. Pulsfront und Phasenfront lassen sich mittels Beugung unter einem Winkel zueinander einrichten.

Aus der Druckschrift WO 2009/111609 A2 ist ein Verfahren zur optischen Kohärenzbildgebung (optical coherence imaging, OCI) bekannt, bei dem auf Methoden der Fourier-Domain Digitalen Holografie (FD-DH) zurückgegriffen wird. Es kann auf streuende, ggf. biologische, Proben angewandt werden und dabei mittels eines Kohärenz-Gating auch Information aus tieferliegenden Probenschichten gewinnen.

Aus der Druckschrift DE 10 2006 015 387 A1 ist eine interferometrische Messvorrichtung für die Vermessung der Oberfläche oder des Volumens eines Messobjektes auf der Basis der Weißlicht-Interferometrie bekannt, durch die das Messobjekt linienhaft mit Licht aus einer kurzkohärenten Lichtquelle beleuchtet wird.

## ZUSAMMENFASSUNG DER ERFINDUNG

Die Aufgabe der Erfindung ist darin zu sehen, ein Verfahren zum Ablichten wenigstens einer Schnittfläche im Innern eines Licht streuenden Objekts vorzuschlagen, das schnell und mit möglichst kostengünstigen Komponenten ausführbar ist, und weiterhin eine Vorrichtung, die die Anwendung des Verfahrens durch ungeschulte Nutzer ermöglicht.

Die Aufgabe wird durch den Gegenstand der unabhängigen Patentansprüche gelöst. Vorteilhafte Ausführungsformen der Erfindung sind Gegenstand der abhängigen Patentansprüche.

Ein Aspekt der Erfindung ist, dass das vom Objekt gestreute Probenlicht auf eine elektronische Kamera abgebildet wird, wobei ein Probenlichtanteil Streuorten auf einer Schnittfläche im Innern des Objekts zugeordnet werden kann. Dieser Probenlichtanteil kann durch eine Verarbeitung des Kamerabildes von den Beiträgen der übrigen Probenlichtanteile separiert werden und führt auf ein Abbild der Schnittfläche, d.h. ein Schnittbild.

Ein weiterer Aspekt der Erfindung besteht darin, dass die Form, Orientierung und Tiefenlage einer abgelichteten Schnittfläche vom Anwender bestimmt werden kann.

Ein weiterer Aspekt der Erfindung ist, dass nacheinander mehrere parallel verlaufende Schnittflächen mit vorbestimmten Abständen zueinander im Innern des Objekts abgelichtet werden können. Eine solche Sequenz von Schnittbildern kann zum Errechnen eines Volumenmodells des Objekts verwendet werden.

Ein weiterer Aspekt der Erfindung besteht in der Anwendung des Verfahrens zur Ablichtung der lebenden Retina. Besonders vorteilhaft ist dabei die Möglichkeit der Erkennung und Kompensation von Relativbewegungen der Retina gegenüber der Kamera während des Erfassens eine Sequenz von Kamerabildern unmittelbar aus den Bilddaten. Dies erlaubt die Ausführung der Erfindung in einem handgehaltenen Messgerät.

Ein erfindungsgemäßes Verfahren zum Ablichten einer Schnittfläche im Innern eines Licht streuenden Objekts umfasst die in Anspruch 1 definierten Schritte.

Eine vorteilhafte Weiterbildung der Erfindung besteht im Verschieben des Weglängenprofils des Referenzstrahls mit einer zeitabhängigen Geschwindigkeit und Erfassen weiterer Kamerabilder jeweils wenigstens indiziert mit einem Maß für die zeitabhängige Verschiebung des Weglängenprofils.

Vorzugsweise wird die Erfindung erweitert um einen Verarbeitungsschritt zum Separieren eines Kamerabildes in ein Schnittbild und ein Auflichtbild basierend auf dem eingerichteten Phasengradienten.

Eine besonders vorteilhafte Ausgestaltung der Erfindung sieht vor, dass ein dreidimensionales Modell des Objekts aus einer Mehrzahl von verschieden indizierten Kamerabildern errechnet wird, indem die Schnittbildwerte einem Voxel-Array zugewiesen werden unter Berücksichtigung der Relativverschiebung von Abbildern identischer Strukturen in verschieden indizierten Auflichtbildern.

Bei der Anwendung der Erfindung auf die lebende Retina als streuendes Objekt ist es eine ganz besonders bevorzugte Ausgestaltung der Erfindung, dass aus der Relativverschiebung des Abbildes wenigstens einer Schichtgrenze der Retina in unterschiedlich indizierten Kamerabildern auf die Änderung des Abstandes der Retina von der Kamera zwischen den Erfassungszeitpunkten der Kamerabilder geschlossen wird.

Die Erfindung gestattet einen dreidimensionalen Retina-Scan innerhalb weniger Sekunden mit einem kostengünstigen und ggf. handgehaltenen Gerät.

## KURZBESCHREIBUNG DER FIGUREN

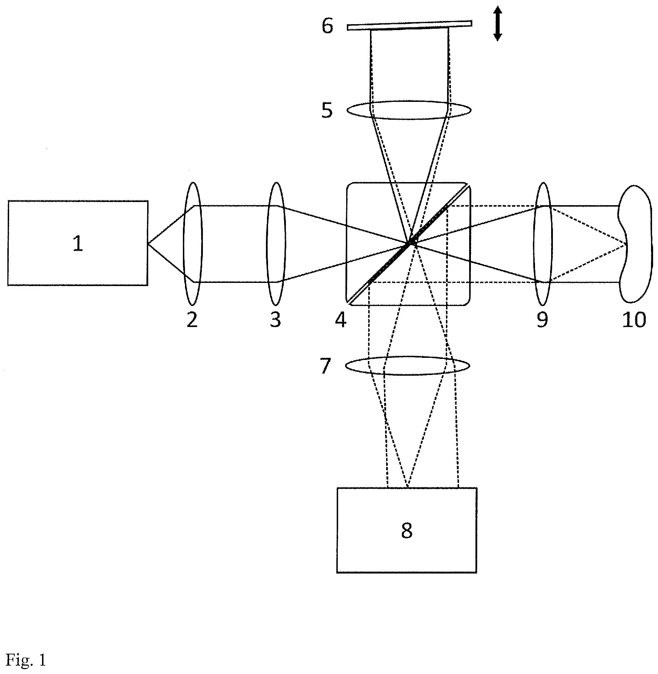

Nachfolgend wird die Erfindung anhand von Ausführungsbeispielen unter Bezug auf Fig. 1 näher beschrieben. Dabei zeigt:

Fig. 1 eine Skizze einer Vorrichtung gemäß einer Ausführungsform der Erfindung.

## DETAILLIERTE BESCHREIBUNG

Es wird zunächst erläutert, welche Relevanz die Größe der Speckle auf der Kamera und der Phasengradient für die Erfindung haben.

Bereits aus der DE 4 108 944 A1 ist bekannt, dass das Probenlicht Speckle aufweist, die problematisch sein können. Ganz allgemein treten Speckle bei der Beleuchtung streuender Objekte mit wenigstens teilkohärentem Licht auf, wenn der mittlere Abstand zwischen den Streuzentren sehr viel kleiner als die Abmessungen des entstehenden Fokusvolumens ist. Bei einer großen Streuerdichte interferieren die an verschiedenen Partikeln eines Objektvolumens gestreuten Anteile daher miteinander, und bei der Abbildung des Objektvolumens auf eine Kamera bilden sich Areale ähnlicher Intensität und Phasenlage aus. Der Kontrast dieser Speckle hängt vom Kohärenzgrad der Lichtquelle und vom optischen Aufbau ab. Die Intensität der Speckle variiert stochastisch verteilt. Die Phasenlage variiert gleichverteilt zwischen Null und $2\pi$.

Die Erfindung sieht nun vor, das Abbilden des vom Objekt gestreuten Probenlichts so vorzunehmen, dass die Speckle entlang wenigstens einer Achse in der Kameraebene einen mittleren Durchmesser D aufweisen, der größer als zwei Pixelbreiten P der Kamera ist. Die Achse kann der Nutzer frei wählen; die Wahl einer der beiden Pixelkoordinatenachsen bietet sich an.

Der mittlere Durchmesser eines Speckle D auf der Kamera kann in an sich bekannter Weise festgelegt werden, denn er entspricht der beugungsbegrenzten Auflösung, die von der Zentralwellenlänge der Lichtquelle $\lambda_0$, dem Durchmesser der Aperturblende B und der Brennweite der Optik f bzw. der numerischen Apertur NA abhängt:

$$D = \frac{2{,}43 * \lambda_0 * f}{B} = \frac{1{,}22 * \lambda_0}{NA}$$

Hieraus ergibt sich, dass die numerische Apertur hinreichend klein einzurichten ist, um zu einem erfindungsgemäßen Speckle-Durchmesser D > 2*P zu gelangen. Die Begrenzung der numerischen Apertur kann auch durch eine zusätzliche Aperturblende erreicht werden, die man in der Fourierebene der Abbildung des Objekts platziert. Entlang der gewählten Achse in der Kameraebene kann dabei auch eine kleinere numerische Apertur eingerichtet werden als in der dazu senkrechten Richtung, indem man eine exzentrische Aperturblende verwendet, vorzugsweise eine rechteckige Apertur mit verschiedenen Seitenlängen. Im Übrigen ist es auch möglich, in der Abbildungsoptik eine zusätzliche vergrößernde Linse vorzusehen, um die Speckle-Durchmesser größer als zwei Pixelbreiten einzurichten.

Die axiale Größe eines Speckle entspricht der Faltung aus der Kohärenzfunktion der Lichtquelle und der axialen Punktspreizfunktion der Abbildung. Für in der OCT übliche numerische Aperturen und Kohärenzlängen wird die axiale Speckle-Größe vornehmlich von der Kohärenzlänge der Lichtquelle bestimmt. Diese Bedingungen gelten auch für die vorliegende Erfindung.

Weiterhin sieht die Erfindung vor, das Referenzlicht dergestalt manipuliert, dass es entlang derselben vorgenannten Achse in der Kameraebene einen Phasengradienten aufweist, d.h. seine Phasenfront soll verkippt gegen die Kameraebene verlaufen. Dies ist durch die Wahl einer Einfallsebene und eines von null verschiedenen Einfallswinkels des Referenzstrahls gegenüber der Normale der Kameraebene zu erreichen. Die Einfallsebene ist dabei so zu wählen, dass sie die Kameraebene entlang jener vorbestimmten Achse schneidet, für die auch der erfindungsgemäße Speckle-Durchmesser D > 2*P vorliegt. Der Einfallswinkel bestimmt den Betrag des Phasengradienten.

Es ist Teil der erfindungsgemäßen Lehre, dass sich die Phase des Referenzlichts um weniger als 2*π über die doppelte Pixelbreite der Kamera und zugleich um mehr als 2*π über den mittleren Durchmesser eines Speckle entlang der vorbestimmten Achse ändern soll.

Der Einfallswinkel $\alpha$ des Referenzstrahls gegenüber der Normale der Kamera erzeugt auf der Kameraebene ein Streifenmuster entlang der Schnittlinie der Kamera mit der Einfallsebene, wenn Referenz- und Probenlicht interferieren. Die Periodenlänge der Streifen beträgt:

$$l_{Fringe} = \frac{\lambda_0}{\sin \alpha}$$

Insbesondere ist es zweckmäßig, das Streifenmuster mit der Kamera abtasten zu können, daher soll die Periodenlänge größer als zwei Pixelbreiten P

$$l_{Fringe} > 2 * P$$

gewählt werden, was zu der Forderung äquivalent ist, dass der Phasengradient kleiner als π/P sein soll. Die Speckle entlang der vorbestimmten Achse sind wiederum mit mindestens einer Periodenlänge des Streifens versehen, wenn der Phasengradient größer ist als 2*π/D.

$$D > l_{Fringe}$$

Dann sind die einzelnen Speckle mit der Modulation des Streifenmusters derart versehen worden, dass die Kamera das Streifenmuster auch in den einzelnen Speckle erfassen kann. Die Speckle sind damit der Separierung der Kamerabilder durch eine Verarbeitung zugänglich, die weiter unten näher beschrieben wird. Denn sowohl die Speckle als auch die Fringes des Streifenmusters treten auf der Kamera nur in Erscheinung, wenn Referenz- und Probenlichtanteile auf den Kamerapixeln dieselbe Weglänge aufweisen und somit kohärent sind. Die sichtbaren Speckle sind insofern den Schnittbildern zuzuordnen.

Anders betrachtet kann die aus der unbekannten Phasenlage von Speckle und Referenzwelle resultierende Uneindeutigkeit der wahren Streustärke mittels des Phasengradienten innerhalb einer einzigen Bildaufzeichnung aufgelöst werden, da zu jedem Speckle auf benachbarten Pixeln der Kamera wenigstens zwei phasenverschobene Messwerte desselben Speckle erfasst werden.

Im Folgenden wird die Festlegung der abgeglichteten Schnittfläche durch die Vorgabe eines Weglängenprofils des Referenzlichts auf den Kamerapixeln näher erklärt.

Der Referenzstrahl definiert beim Einfall auf die Kamera ein Weglängenprofil auf den Pixeln entlang der Achse mit dem Phasengradienten. Das vom Objekt gestreute Probenlicht enthält womöglich einen Anteil, der genau diesem Weglängenprofil entspricht, und nur dieser Anteil trägt dann zu Interferenzmustern im Kamerabild bei. Die abgebildeten Objektpunkte, deren Streulicht das besagte Weglängenprofil aufweist, liegen auf einer Schnittfläche im Innern des streuenden Objekts. In der Regel handelt es sich bei der Schnittfläche um eine Ebene, deren Normale um den Winkel $\alpha$ - den Einfallswinkel des Referenzstrahls auf die Kamera - gegen die optische Achse verkippt ist.

Das Weglängenprofil auf der Kamera hängt direkt von der Weglängenverteilung im Strahlquerschnitt des Referenzstrahls ab, wenn dieser auf die Kameraebene trifft. Wird das Referenzlicht über Spiegel oder Prismen abgelenkt, so dass es schräg einfällt, dann stimmen Phasenfronten und Pulsfronten des Lichts überein, d.h. im Strahlquerschnitt parallel zur Phasenfront ist die Weglänge überall dieselbe. Es stellt sich aber auf der Kamera ein lineares Weglängenprofil ein, das beispielsweise ein Weglängeninterval mit einer Intervallbreite von etwa 500 Wellenlängen $\lambda_0$ umfasst, also typisch einige Hundert Mikrometer. Streng genommen ist das Weglängenprofil auf der Kamera eine Funktion der beiden Pixelkoordinaten, aber es variiert hier nur entlang einer Achse, die von der Einfallsebene des Referenzlichts vorbestimmt ist.

Das Licht aus irgendeinem Objektpunkt wird in einen Kamerapixel fokussiert und führt zur Interferenz mit dem Referenzlicht, wenn der optische Abstand des Objektpunktes zur Kamera genau dem auf diesem Pixel vorliegenden Wert des Weglängenprofils entspricht. Dann und nur dann liegt der Objektpunkt in der Schnittfläche. Deshalb stammen die interferenzfähigen Probenlichtanteile in einem Kamerabild gewöhnlich aus verschiedenen Objekttiefen, d.h. die abgebildete Schnittfläche liegt schräg zur optischen Achse. Die Lage der Schnittfläche wird generell vom Weglängenprofil auf der Kamera bestimmt.

Es ist aber durch Beugung an einem geeigneten Gitter auch möglich, wie schon im Stand der Technik angesprochen, das Weglängenprofil des Referenzlichts trotz des gewinkelten Lichteinfalls auf die Kamera so einzurichten, dass auf allen Pixeln beispielsweise dieselbe Weglänge vorliegt. In diesem Fall liegt die Schnittfläche senkrecht zur optischen Achse der Abbildung.

Nebenbei soll noch erwähnt werden, dass man auch mit sphärischen Phasenfronten und Pulsfronten, d.h. mit Licht aus einer Punktquelle, zum Ziel gelangt. Das Weglängenprofil auf der Kamera hat dann aber einen komplizierteren Verlauf, so dass die abgebildeten Schnittflächen im Objekt gekrümmte Flächen sind. In dieser Beschreibung wird allein zur Vereinfachung angenommen, dass das Licht durch ebene Wellen beschrieben ist.

Jedenfalls ist hervorzuheben, dass das Weglängenprofil des Referenzlichts auf der Kamera vom Nutzer vorgegeben werden kann. Dieses Profil legt fest, welche Schnittfläche des Objekts - hinsichtlich Orientierung, Form und Abstand zur Kamera - dann zu Interferenzen im erfassten Kamerabild beitragen kann.

Das erfindungsgemäße Ablichten erzeugt speziell strukturierte Kamerabilder. Die Strukturen der Bilder enthalten u. a. Interferenzmuster, die dazu geeignet sind, aus vorbestimmten Schnittflächen stammende Lichtanteile von anderen Lichtanteilen zu separieren. Die Erfindung kann verstanden werden als ein Verfahren zur Erzeugung schneller Fotografien von inneren Schnittflächen eines Licht streuenden Objekts mit Kurzkohärenzcodierter Lagedefinition der Schnittflächen ("Short-Coherence Interior Snapshot of Scattering Objects in Reflection", SCISSOR).

Eine tatsächliche Darstellung der Schnittflächen kann aus dem erfassten Kamerabild mit einer sehr einfachen Bildverarbeitung gewonnen werden, die weiter unten beschrieben wird.

Eine ganz besonders bevorzugte Ausgestaltung der Erfindung besteht im Verschieben des Weglängenprofils des Referenzstrahls mit einer zeitabhängigen Geschwindigkeit und Erfassen weiterer Kamerabilder jeweils wenigstens indiziert mit einem Maß für die zeitabhängige Verschiebung des Weglängenprofils. Auf diese Weise werden Ablichtungen von einer Mehrzahl von zueinander parallel liegenden Schnittflächen im Innern des Objekts gewonnen.

Im einfachsten Fall erfolgt die Verschiebung des Weglängenprofils - dies bedeutet, alle Weglängen auf den Kamerapixeln werden gleichzeitig um denselben Betrag verändert - mit einer zeitabhängigen Geschwindigkeit, die einen Rechteckverlauf besitzt. Beispielsweise werden alle Weglängen monoton vergrößert mit zwei sich abwechselnden, unterschiedlich großen Geschwindigkeiten. Während eines Zeitintervalls, in dem die kleinere Geschwindigkeit - z.B. mit Wert null - vorliegt, erfolgt die Erfassung eines Kamerabildes. Das nachfolgende Kamerabild wird erfasst, nachdem wenigstens ein Zeitintervall mit der größeren Geschwindigkeit verstrichen ist und wieder die kleinere Geschwindigkeit vorliegt. Die Kamerabilder lichten so parallel liegende Schnittflächen ab, und der Abstand dieser Schnittflächen ist durch die Verschiebung beschrieben. Dabei reicht es auch aus, einen Zeitindex zu protokolieren und die zeitabhängige Geschwindigkeit zu kennen, um die Verschiebung später zu errechnen.

Die Verschiebung des Weglängenprofils auf der Kamera kann durchaus über einen großen Wertebereich in der Größenordnung Zentimeter erfolgen. Die jeweils abgelichtete Schnittfläche wird dabei Bild für Bild durch das Objekt hindurch geschoben, und man erzielt in der Zusammenschau aller Kamerabilder eine komplett rekonstruierbare, dreidimensionale Erfassung der Streustärke im Objektinnern, wenn man den Abstand benachbarter Schnittflächen in der Größenordnung der Kohärenzlänge der Lichtquelle wählt. Die Erfindung erlaubt somit die Ablichtung auch eines Volumens des streuenden Objekts.

Zur Ausführung der Erfindung werden folgende Parameterbereiche empfohlen:

Die Zentralwellenlänge $\lambda_0$ wird vorzugsweise aus dem nahen bis mittleren Infrarotspektrum gewählt, besonders bevorzugt beträgt sie zwischen 700 und 1500 Nanometer.

Die numerische Apertur NA liegt vorzugsweise im Intervall von 0,01 bis 0,1. Besonders bevorzugt beträgt die numerische Apertur zwischen 0,02 und 0,05.

Die Pixelbreite P der Kamera liegt vorzugsweise im Intervall von 0,5 bis 20 Mikrometer. Besonders bevorzugt beträgt sie zwischen 1 und 5 Mikrometer.

Ein einzelnes erfasstes Kamerabild kann vorzugsweise mittels einer zweidimensionalen Fourier-Filterung in ein Schnittbild der abgelichteten Schnittfläche und in ein Auflichtbild des Objekts separiert werden. Die erfassten Lichtanteile, die dabei dem Schnittbild zuzuordnen sind, sind nunmehr mit einer vorbekannten periodischen Intensitätsmodulation versehen, die sich aus dem eingerichteten Phasengradienten entlang der Kamera ergibt. Zwar sind diese Lichtanteile von Speckle gekennzeichnet, aber auch die einzelnen Speckle tragen - für die Kamera gut erkennbar - die besagte Modulation. Die Modulation wird durch einen vorbekannten zweidimensionalen Wellenzahlvektor beschrieben, der auch häufig als die Raumfrequenz der Modulation bezeichnet wird.

Eine zweidimensionale Fourier-Transformation eines Kamerabildes führt insbesondere auf Fourier-Komponenten in der Umgebung dieser Raumfrequenz. Diese Fourier-Komponenten können separat ermittelt, hiernach um die Raumfrequenz des Phasengradienten in den Ursprung verschoben und in den Ortsraum zurücktransformiert werden, um eine bildliche Darstellung allein jener Schnittfläche zu erzeugen, die zur Interferenz im Kamerabild beigetragen hat. Die Fourier-Rücktransformation aller übrigen Fourier-Komponenten des Kamerabildes führt auf ein herkömmliches Auflichtbild des Objekts, das keine Interferenzmuster durch die Überlagerung mit dem Referenzstrahl zeigt.

Unter Fourier-Filterung wird hier also verstanden, dass eine Fourier-Transformation vom Ortsraum in den Wellenzahlraum und zurück erfolgt, bei der vorbestimmte Fourier-Koeffizienten selektiert und separiert werden. Dieses Vorgehen zum Filtern von frequenzgebundenen Informationen ist dem Fachmann aus der Signalverarbeitung geläufig.

Das vorbeschriebene Separieren eines Kamerabildes in ein Schnittbild und ein Auflichtbild durch zweidimensionale Fourier-Filterung des erfassten Kamerabildes bezüglich des eingerichteten Phasengradienten führt vorteilhafterweise automatisch dazu, dass erkennbare Strukturen in den separierten Bildern gemeinsam registriert sind. Dies bedeutet insbesondere, dass alle Positionen von Strukturen im Auflichtbild zur Positionsbestimmung von Strukturen im Schnittbild herangezogen werden können.

Dies ist vor allem dann vorteilhaft, wenn man mit einem handgehaltenen SCISSOR-System die Ablichtung eines Objektvolumens, beispielsweise der Retina, durchführen möchte. Dazu muss man eine Sequenz von Kamerabildern für verschiedene Schnittflächen des Objekts erfassen, um aus den Schnittbildern auf die dreidimensionale Verteilung der Streustärken des Objekts zu schließen. Diese Verteilung kann beispielsweise durch ein numerisches Voxel-Array als Modell des Objekts repräsentiert werden, wobei man den dreidimensional indizierten Voxeln die Schnittbildwerte - interpretiert als die zu entnehmenden Streustärken - passgenau zuordnet.

Da die Kamerabilder mit einem Maß für die Verschiebung des Weglängenprofils des Referenzstrahls auf der Kamera indiziert sind, steht mit der Bildsequenz bereits eine Koordinate zur Anordnung der verschiedenen Schnittbilder in einem Voxel-Array zur Verfügung. Weil sich aber Kamera und Objekt während der Messdauer - dem Erfassen der gesamten Bildsequenz - durch Anwender- oder Objektbewegungen gegeneinander verschieben können, können auch die Schnittbilder gegeneinander verschoben sein. Die nacheinander erfassten Auflichtbilder sind jedoch in Abwesenheit jeder Anwenderbewegung praktisch identisch. Sie zeigen alle die Strukturen, die man mit einer herkömmlichen Kamera auch sehen würde. Zum automatischen Ermitteln von Verschiebungen identischer Strukturen in elektronischen Bildern kennt der Stand der Technik effiziente Algorithmen, beispielsweise kann über den Verschiebungsvektor in einem Hough-Raum abgestimmt werden.

Es ist deshalb eine bevorzugte Ausgestaltung der Erfindung, dass ein dreidimensionales Modell des Objekts aus einer Mehrzahl von verschieden indizierten Kamerabildern errechnet wird, indem die Schnittbildwerte einem Voxel-Array zugewiesen werden unter Berücksichtigung der Relativverschiebung von Abbildern identischer Strukturen in verschieden indizierten Auflichtbildern. Die Erzeugung solcher dreidimensionaler Modelle bzw. Voxel-Arrays ist schon deshalb vorteilhaft, weil sie das numerische Errechnen weiterer beliebig orientierter Schnittbilder durch das Objekt in an sich bekannter Weise erlauben.

Wenn von dem abzulichtenden Objekt weiterhin vorbekannt ist, dass es wenigstens eine streuende Schichtgrenze mit vorbekannter Orientierung zur optischen Achse aufweist - dies ist insbesondere bei der lebenden Retina der Fall, wo solche Grenzen zwischen Zellschichten vorliegen -, dann erweist es sich unerwartet als vorteilhaft, wenn die erfindungsgemäß abgelichteten Schnittflächen im Objektinnern gegen die optische Achse verkippt liegen. Denn die Schnittbilder zeigen einen gut erkennbaren Horizont, d.h. ein Abbild der Schnittlinie zwischen den Schnittflächen und der besagten streuenden Schichtgrenze, für alle Schnittflächen, für die diese Schnittlinie existiert. Der Horizont "bewegt" sich durch die Schnittbilder, wenn sich die Verschiebung des Weglängenprofils ändert, in dem Sinne, dass er üblich in jedem Schnittbild bei anderen Pixelkoordinaten zu sehen ist. Der Horizont bewegt sich aber darüber hinaus auch durch tatsächliche Eigenbewegungen des Anwenders.

Die Anwenderbewegung zwischen den Aufnahmen der Kamerabilder kann wie beschrieben mit Hilfe der Auflichtbilder in der Ebene senkrecht zur optischen Achse, also lateral, kompensiert werden. Die dritte Komponente der Anwenderbewegung in Richtung der optischen Achse ist normalerweise für OCT-Systeme unzugänglich. Doch hier kann sie auch genau bestimmt werden, weil man auch nach der Kompensation der lateralen Anwenderbewegungen immer noch Bewegungen des Horizontes ausmacht, wenn diese von einer Änderung der Schnittfläche herrühren, die der Anwender durch Abstandsänderungen herbeiführt. Weil die Schnittflächen verkippt gegen die streuende Schichtgrenze liegen, bewegt sich die abgebildete Schnittlinie dann zwangsläufig lateral und wird dabei von der Kamera erfasst. Dies gestattet speziell bei der Retina-Messung, dass aus der Relativverschiebung des Abbildes wenigstens einer Schichtgrenze der Retina in unterschiedlich indizierten Kamerabildern auf die Änderung des Abstandes der Retina von der Kamera zwischen den Erfassungszeitpunkten der Kamerabilder geschlossen wird.

Es ist somit im Rahmen der Erfindung auch ohne zusätzlichen Messaufwand oder Sensorik, sondern vielmehr direkt aus den Bilddaten der erfassten Kamerabilder, möglich, dreidimensionale Modelle des streuenden Objekts zu gewinnen, in denen Anwenderbewegungen in drei Dimensionen kompensiert worden sind.

In Fig. 1 ist die Skizze einer exemplarischen SCISSOR-Vorrichtung nach Art eines Michelson-Interferometers dargestellt.

Aus einer zeitlich kurzkohärenten Lichtquelle (1) wird divergent austretendes Licht zunächst mittels einer Kollimatorlinse (2) kollimiert. Ein Strahlteiler (4) mit einem halbdurchlässigen Spiegel ist von vier Linsen (3, 5, 7, 9) umgeben, die so angeordnet sind, dass sie zum einen den Referenzspiegel (6) und das Objekt (10) jeweils mit kollimiertem Licht beleuchten und zum anderen das Objekt (10) auf die Kamera (8) abbilden. Der Referenzspiegel (6) ist um einen vorbestimmten Winkel $\alpha$ verkippt, wodurch der reflektierte Referenzstrahl geringfügig seitlich abgelenkt wird und leicht schräg auf die Ebene der elektronischen Kamera (8) trifft. Das Licht streuende Objekt (10) ist in der Fokusebene der Objektivlinse (9) angeordnet, wobei die numerische Apertur in der Skizze zur Verdeutlichung überhöht gezeichnet ist. Tatsächlich werden alle Licht streuenden Punkte des Objekts (10) mehr oder weniger scharf auf die Kamera (8) abgebildet unabhängig von ihrer Tiefenlage im Objekt (10). Der Abstand des Referenzspiegels (6) zum Strahlteiler (4) wird durch einen nicht dargestellten Stellantrieb variiert - angedeutet durch den Doppelpfeil in der Skizze.

Das Winkelintervall für den Winkel $\alpha$ ergibt sich aus den bereits dargelegten Überlegungen zu

$$M * D = M * \frac{1{,}22 * \lambda_0}{NA} > \frac{\lambda_0}{\sin \alpha} > 2 * P$$

Wenn man die Ungleichung durch $\lambda_0$ dividiert und den Kehrwert bildet:

$$\frac{NA}{1{,}22 * M} < \sin \alpha < \frac{\lambda_0}{2 * P}$$

Der Vergrößerungsfaktor M ist das Verhältnis von Bildgröße zu Objektgröße bei der Abbildung. Er wird hier explizit eingeführt, um den Gebrauch einer vergrößernden Linse in der Abbildungsoptik z.B. bei Wahl einer zu großen NA, einzuschließen. Wichtig für die Erfindung ist der Speckle-Durchmesser auf der Kamera, der hier M*D beträgt. Gewöhnlich kann man es bei M = 1 belassen.

Beispielsweise liegt der zulässige Winkelbereich für $\alpha$ zwischen 2,35° und 4,59°, wenn die Kamera Pixel der Pixelbreite 5 Mikrometer aufweist, die Zentralwellenlänge bei 800 Nanometer gewählt wird und die NA den in der OCT gängigen Wert von 0,05 beträgt. Das Winkelintervall wird größer für kleinere numerische Aperturen und/oder größere Wellenlängen. Nur relativ große Kamerapixel dürften eine Vergrößerung erfordern.

Eine SCISSOR-Vorrichtung ist immer eine Interferometer-Vorrichtung mit Proben- und Referenzarm. Dabei ist die Skizze in Fig. 1 nur als eine exemplarische Ausführung der Vorrichtung zu verstehen. Beispielsweise muss das Referenzlicht vom Referenzspiegel (6) nicht zwingend erneut durch den Strahlteiler (4) und die Linsen (5, 7) geführt werden, sondern könnte auch an diesen Komponenten vorbei auf die Kamera (8) gelangen. Auch der verkippte Referenzspiegel (4) ist nur bevorzugt, aber nicht zwingend erforderlich. Erforderlich ist ein Phasengradient auf der Kamera (8), der vorzugsweise einfach durch den gewinkelten Einfall des Referenzlichts erzeugt wird. Beispielsweise könnte alternativ das Referenzlicht seitlich versetzt und senkrecht zur Kamera (8) durch ein Beugungsgitter geführt werden, wobei die Kamera (8) an einem Ort in Richtung auf ein Nebenbeugungsmaximum des Referenzlichts angeordnet ist. In diesem Fall fällt nur ein Teil des Referenzlichts - aber wieder unter einem Winkel - auf die Kamera (8) ein. Der Typ des Interferometers ist auch nicht auf den Michelson-Aufbau beschränkt.

Allgemeiner formuliert handelt es sich um eine Interferometer-Vorrichtung zur Ablichtung eines Volumens eines streuenden Objekts aufweisend eine Lichtquelle (1), die Licht mit Kohärenzlänge kleiner als 25 Mikrometer und Zentralwellenlänge $\lambda_0$ emittiert, einen Strahlteiler (4) zur Aufteilung des Lichts in Proben- und Referenzarm, Mittel (5, 6) zum Verändern der Referenzarmlänge, Mittel (9) zur flächigen Beleuchtung des Objekts (10) im Probenarm, eine elektronische Kamera (8) mit Pixeln der Breite P, eine Abbildungsoptik (7, 9) mit numerischer Apertur NA und Vergrößerung M angeordnet zur Abbildung des vom Objekt (10) gestreuten Lichts auf die Kamera (8) und eine Recheneinheit zur Verarbeitung der erfassten Kamerabilder, dadurch gekennzeichnet, dass der Einfallswinkel $\alpha$ des Lichts aus dem Referenzarm auf die Kamera gemäß der Bedingung $\lambda_0/(2*P) > \sin(\alpha) > NA/(1{,}22*M)$ eingerichtet ist.

Eine vorteilhafte Ausgestaltung der Vorrichtung kann darin bestehen, dass die Abbildungsoptik (7, 9) eine exzentrische Aperturblende umfasst, wie weiter oben erläutert wird.

Vorzugsweise weist die Vorrichtung einen auf die Mittel (5, 6) zum Verändern der Referenzarmlänge wirkenden Stellantrieb und eine Stellantriebsteuerung auf. Die Stellantriebsteuerung soll mit der Recheneinheit zur Verarbeitung der erfassten Bilder kommunizierend verbunden sein, um der Recheneinheit Daten über den Zustand des Referenzarms, z.B. seiner Länge und/oder seiner Längenänderungsgeschwindigkeit, wenigstens mit der Bilderfassung zeitlich Schritt haltend zu übermitteln. Da die Änderung der Referenzarmlänge zugleich die Verschiebung des Weglängenprofils des Referenzstrahls auf der Kamera (8) bewirkt, kann die Recheneinheit die Daten der Stellantriebsteuerung zur Indizierung der Kamerabilder verwenden, z.B. beides zusammen in den Datenspeicher der Kamera (8) schreiben.

Weiterhin vorzugsweise ist die Recheneinheit dazu ausgebildet, wenigstens vorbestimmte zweidimensionale Fourier-Koeffizienten der erfassten Kamerabilder zu errechnen. Sie kann dadurch die Separierung der erfassten Kamerabilder in Schnittbilder und Auflichtbilder durchführen und diese als getrennte Bilder zum Beispiel im elektronischen Datenspeicher der Kamera (8) ablegen.

Alternativ kann die Recheneinheit aber auch darauf beschränkt sein, nur das Vorhandensein eines auswertbaren Schnittbildes im Kamerabild zu überprüfen. Dazu kann es ausreichen, gezielt nur Fourier-Komponenten im Bereich der vorbekannten Raumfrequenz des Phasengradienten aus den Kamerabildern zu errechnen und zu überprüfen, ob deren Absolutbeträge vorbestimmte Schwellenwerte übersteigen. Ist dies nicht der Fall, so ist kein auswertbares Schnittbild erfasst worden. Die wahrscheinliche Ursache liegt darin, dass die Referenzarmlänge erheblich vom Abstand des Objekts zur Kamera abweicht, so dass keine Interferenz mit Probenlicht möglich ist. Besonders bei der Vermessung der Retina des lebenden Auges ist dieser Fall zunächst wahrscheinlich, wenn die Vorrichtung vor das Auge gehalten wird. Das Gerät muss die Lage der Retina erst finden und den Referenzarm darauf einstellen. Die Recheneinheit kann eine solche Situation erkennen und den Stellantrieb ihrerseits veranlassen, die Referenzarmlänge in großen Schritten, z.B. einige Hundert Mikrometer bis hin zu Millimeter, zu ändern, um das Auffinden des Objekts zu beschleunigen.

Jedenfalls ist es eine bevorzugte Ausgestaltung, dass die Recheneinheit dazu ausgebildet ist, Fourier-Koeffizienten von erfassten Kamerabilder mit der Bilderfassung zeitlich Schritt haltend zu ermitteln und mit vorbestimmten Schwellenwerten zu vergleichen, und anhand des Vergleichsergebnisses vorbestimmte Befehle an die Stellantriebsteuerung zu senden. Dieses Konzept der Rückkopplung der Bilderfassung und -verarbeitung auf die Stellantriebsteuerung erlaubt insbesondere auch eine befundabhängige Variation der Änderung des Weglängenprofils, mithin der Scangeschwindigkeit über die Schnittflächen.

Vorzugsweise erfolgt die Änderung des Weglängenprofils des Referenzstrahls während einer Messung monoton auf- oder absteigend über einen Bereich von wenigstens einem Zentimeter, besonders bevorzugt 1 bis 2 Zentimeter. Dabei liegt es im Rahmen der Erfindung, mehrere Messungen desselben Objekts unmittelbar nacheinander vorzunehmen und die Ergebnisse einer früheren Messung auch für Befehle an die Stellantriebsteuerung bei nachfolgenden Messungen zu verwenden.

Beispielsweise bei der Retinamessung kann in einer ersten Messung mit großer Schrittweite der Änderung des Weglängenprofils - z.B. um 100 Mikrometer - die Position der Retina in einem Messfenster - etwa der Breite 1-2 Zentimeter - bestimmt werden. Ist die Position der Retina ermittelt, dann werden der axiale Messbereich und die Schrittweite deutlich verkleinert, beispielsweise jeweils um eine Größenordnung. Die genaue axiale Position des Messfensters kann an die zuvor gemessene Position der Retina angepasst werden, und es erfolgt unverzüglich eine zweite Messung mit der verkleinerten Schrittweite.

Es ist hier zu betonen, dass der Stellantrieb sehr preisgünstig sein kann, weil er keinen hohen technischen Ansprüchen genügen muss. Beispielsweise kann der Referenzspiegel (6) schrittweise bewegt werden, wobei er während einer Bilderfassung der Kamera still steht. Der Stellantrieb kann den Referenzspiegel (4) aber auch kontinuierlich bewegen, während die Kamera (8) eine Sequenz von Bildern erfasst. Dies ist gerade bei hohen Bildraten der Kamera besonders vorteilhaft. Dabei kann die Geschwindigkeit der Bewegung während der Messdauer durch die Stellantriebsteuerung kontrolliert variiert werden, sei es programmgesteuert nach Vorgaben oder durch Befehle der Recheneinheit.

Heute günstig erhältliche Kameras können z.B. etwa 800 Bilder pro Sekunde aufnehmen, d.h. ein Bild alle 1250 Mikrosekunden. Für eine im Sinne des Abtasttheorems vollständige Abtastung eines Volumens im Objekt sollte sich in dieser Zeitspanne die Weglänge des Referenzlichts um höchsten die halbe Kohärenzlänge ändern, z.B. typisch um 7,5 Mikrometer. Die maximale Weglängenänderungsgeschwindigkeit darf dann $v_R$= 6 mm/s betragen, bzw. im obigen Beispiel darf der Referenzspiegel (6) um maximal 3 mm/s verschoben werden. Vorteilhafterweise beträgt die Messdauer somit für einen Messbereich von z.B. 1,8 Zentimeter nicht mehr als 3 Sekunden.

Will man die Referenzarmlänge allerdings kontinuierlich, d.h. auch während der Erfassung einzelner Kamerabilder, verändern, so muss man auf eine ausreichend kurze Belichtungszeit achten, weil alle zur Interferenz beitragenden Lichtanteile ausgelöscht werden, wenn sich die Phase der Referenzwelle während der Integration auf allen Pixeln der Kamera (8) um $2\pi$ ändert,

was bei einer Änderung des Weglängenprofils um eine Zentralwellenlänge $\lambda_0$ der Fall ist. Deshalb ist die Belichtungszeit kleiner einzurichten als $\lambda_0 / v_R$. Dies gestattet, dass das Weglängenprofil des Referenzstrahls kontinuierlich verschoben wird, wobei sich alle Weglängen während einer Belichtungszeit der Kamera um höchstens einen vorbestimmten Bruchteil der Zentralwellenlänge ändern.

Mit den bisherigen Beispielwerten $\lambda_0 = 800$ nm und $v_R = 6$ mm/s ist eine Belichtungszeit kürzer als etwa 133 Mikrosekunden vorzusehen. Vorzugsweise wird man die Belichtungszeit kürzer als $\lambda_0 / (2{*}v_R)$, besonders bevorzugt etwa zu $\lambda_0 / (4{*}v_R)$ wählen. In jedem Fall ist die Belichtungszeit mit weniger als 200 Mikrosekunden zur Vermeidung kurzzeitiger Bewegungsartefakte in den Einzelbildern geeignet.

Zu guter Letzt, soweit die Messdaten der Vorrichtung beim Nutzer zu Hause erhoben werden - oder an irgendeinem anderen Ort, an dem sich nicht die Person befindet, die die Daten analysieren soll - ist eine bevorzugte Ausführungsform der Vorrichtung gekennzeichnet durch eine Datenschnittstelle, die drahtlos etwa über WLAN oder über Mobilfunknetz die aufgenommenen und ggf. bearbeiteten Bilder vorzugsweise automatisch an vorbestimmte Empfänger versendet. Die Empfänger können Smartphones von Ärzten sein oder auch Server im Internet, die zusätzliche Bearbeitungen und/oder Archivierungen durchführen. Beispielsweise können so Gewebeveränderungen zwischen zwei Messungen der Retina auch automatisch in Differenzbilder übersetzt und an den Arzt ausgeliefert werden.

Wesentliche Vorteile einer SCISSOR-Vorrichtung sind:

Das Gerät ist kompakt herzustellen mit ab Werk fixierten Bauteilen. Der Anwender hat keinerlei Einstellungen selbst vorzunehmen. Die Referenzarmlänge wird durch eine programmgemäße Steuerung automatisch variiert. Die Lage bzw. der Abstand der Retina zum Messgerät ist a priori unbekannt und wird während der Messung auch nicht notwendig absolut ermittelt. Vielmehr kann die Variation der Referenzarmlänge über einen so großen Messbereich erfolgen, dass die Retina des Anwenders zweifelsfrei in diesem Messbereich liegt.

Das Gerät kann vom Patienten oder von Pflegepersonal ohne OCT-Vorkenntnisse vor das zu untersuchende Auge gehalten werden, solange es nur möglichst ruhig gehalten wird.

Die Messung benötigt nur wenige Sekunden, in denen das ruhige Halten auch älteren Patienten noch gut gelingen sollte. Bewegungen des Anwenders werden überdies anhand der Auflichtbilder erkannt und können bei einer numerischen Modellierung kompensiert werden.

Die absehbar teuerste Komponente der SCISSOR-Vorrichtung ist die elektronische Kamera, die eine möglichst hohe Bildrate besitzen soll. Alle übrigen Komponenten sind ausgesprochen preiswert erhältlich. Das Gerät sollte in einer für den Privathaushalt vertretbaren Preisklasse verkäuflich sein.

Eine gespeicherte Bildsequenz kann mittels Datenfernübertragung zum Arzt gelangen. Bevorzugt wird sie - ggf. anonymisiert - an einen Server im Internet geschickt, der eine automatische Datenaufbereitung vornimmt. Diese kann beispielsweise das Erstellen eines dreidimensionalen Modells der Retina mit vom Arzt geforderten Schnittbildern umfassen. Es kann insbesondere auch eine Archivierung der Dateien erfolgen. Ferner ergibt sich für Verlaufskontrollen hieraus die Möglichkeit, Veränderungen der Streustärkenverteilung in der Retina mittels Vergleich von Bildsequenzen und/oder Modellen zwischen wenigstens zwei Erfassungszeitpunkten darzustellen.

Die Verfügbarkeit einer großen Anzahl von Retina-Abbildungen und Therapie-Verlaufsaufzeichnungen im Internet kann Zwecken von medizinischer Forschung und Lehre dienen.

Ein SCISSOR-Gerät kann im Übrigen nicht nur medizinischen, sondern auch sicherheitstechnischen Zielsetzungen dienen. Denn ist die Zuordnung insbesondere von Retina-Scans zu bestimmten Personen möglich und sogar beabsichtigt, so kann deren Identität anhand der eindeutigen und jetzt auch dreidimensional erfassbaren Retina-Strukturen zuverlässig biometrisch verifiziert werden.

**Patentansprüche**

1. Verfahren zum Ablichten einer Schnittfläche im Innern eines Licht streuenden Objekts (10) mit den Schritten:

   Bereitstellen einer Lichtquelle (1), die Licht mit einer vorbestimmten Zentralwellenlänge und einer Kohärenzlänge kleiner als 25 Mikrometer emittiert;
   Aufteilen des Lichts der Lichtquelle (1) in Probenlicht und Referenzlicht;
   flächiges Beleuchten des Objekts (10) mit dem Probenlicht;
   Zuführen des vom Objekt (10) gestreuten Probenlichts zu einer elektronischen Kamera (8) mit Pixeln der Pixelbreite P;
   wobei das vom Objekt (10) gestreute Probenlicht der elektronischen Kamera so zugeführt wird, dass das Objekt (10) auf der elektronischen Kamera (8) abgebildet wird, unter Einrichten eines mittleren Speckle-Durchmessers D größer als zwei Pixelbreiten entlang wenigstens einer Achse in der Kameraebene; und
   Interferieren lassen von Referenzlicht und Probenlicht auf der Kamera (8) unter

   Einrichten eines Weglängenprofils und eines Phasengradienten des Referenzlichts entlang der vorbestimmten Achse in der Kameraebene, wobei der Phasengradient einen Betrag aus dem Intervall zwischen $2\pi/D$ und $\pi/P$ aufweist, und

Erfassen eines Kamerabildes, wobei nur das von einer Schnittfläche im Innern des Objekts (10) gestreute und das Weglängenprofil des Referenzlichts aufweisende Probenlicht zu Interferenzmustern beiträgt.

2. Verfahren nach Anspruch 1, **gekennzeichnet durch** Verschieben des Weglängenprofils des Referenzstrahls mit einer zeitabhängigen Geschwindigkeit und Erfassen weiterer Kamerabilder jeweils wenigstens indiziert mit einem Maß für die zeitabhängige Verschiebung des Weglängenprofils.

3. Verfahren nach Anspruch 2, **dadurch gekennzeichnet, dass** das Weglängenprofil des Referenzstrahls kontinuierlich verschoben wird, wobei sich alle Weglängen während einer Belichtungszeit der Kamera (8) um höchstens einen vorbestimmten Bruchteil der Zentralwellenlänge ändern.

4. Verfahren nach einem der vorangehenden Ansprüche, **gekennzeichnet durch** Separieren eines Kamerabildes in ein Schnittbild und ein Auflichtbild basierend auf dem eingerichteten Phasengradienten.

5. Verfahren nach Anspruch 4, wobei das Separieren des Kamerabildes eine zweidimensionale Fourier-Filterung des erfassten Kamerabildes bezüglich des eingerichteten Phasengradienten beinhaltet.

6. Verfahren nach Anspruch 4 oder 5 und nach Anspruch 2, **dadurch gekennzeichnet, dass** ein dreidimensionales Modell des Objekts (10) aus einer Mehrzahl von verschieden indizierten Kamerabildern errechnet wird, indem die Schnittbildwerte einem Voxel-Array zugewiesen werden unter Berücksichtigung der Relativverschiebung von Abbildern identischer Strukturen in verschieden indizierten Auflichtbildern.

7. Verfahren nach einem der vorangehenden Ansprüche, **dadurch gekennzeichnet, dass** das Licht streuende Objekt (10) die Retina eines lebenden Auges ist.

8. Verfahren nach Anspruch 7, **dadurch gekennzeichnet, dass** die Schnittfläche eine die Zellschichten der Retina unter einem Winkel schneidende Ebene ist.

9. Verfahren nach Anspruch 2 und Anspruch 8, **dadurch gekennzeichnet, dass** aus der Relativerschiebung des Abbildes wenigstens einer Schichtgrenze der Retina in unterschiedlich indizierten Kamerabildern auf die Änderung des Abstandes der Retina von der Kamera (8) zwischen den Erfassungszeitpunkten der Kamerabilder geschlossen wird.

10. Verfahren nach einem der Ansprüche 7 bis 9, **gekennzeichnet durch** Darstellen von Veränderungen der Retina anhand des Vergleichs von Kamerabildern und/oder Modellen mit archivierten Kamerabildern und/oder Modellen wenigstens eines früheren Erfassungszeitpunktes.

11. Verfahren nach einem der Ansprüche 7 bis 10, **gekennzeichnet durch** biometrische Verifikation der Identität eines Nutzers.

12. Interferometer-Vorrichtung zur Ablichtung eines Volumens eines streuenden Objekts aufweisend:

   eine Lichtquelle (1), die Licht mit Kohärenzlänge kleiner als 25 Mikrometer und Zentralwellenlänge $\lambda_0$ emittiert,
   einen Strahlteiler (4) zur Aufteilung des Lichts in Proben- und Referenzarm,
   Mittel (5, 6) zum Verändern der Referenzarmlänge,
   Mittel (9) zur flächigen Beleuchtung des Objekts (10) im Probenarm,
   eine elektronische Kamera (8) mit Pixeln der Breite P,
   eine Recheneinheit zur Verarbeitung der erfassten Kamerabilder,
   eine Abbildungsoptik (7, 9) mit numerischer Apertur NA und Vergrößerung M, angeordnet zur Zuführung des vom Objekt (10) gestreuten Lichts zu der Kamera (8), wobei die Abbildungsoptik (7,9) so eingerichtet ist, dass sie das Objekt (10) auf der Kamera (8) abbildet;
   wobei der Einfallswinkel $\alpha$ des Lichts aus dem Referenzarm auf die Kamera (8) gemäß der Bedingung $\lambda_0/(2{*}P) > \sin(\alpha) > NA/(1{,}22{*}M)$ eingerichtet ist.

13. Interferometer-Vorrichtung nach Anspruch 12, **dadurch gekennzeichnet, dass** die Abbildungsoptik (7, 9) eine exzentrische Aperturblende umfasst.

14. Interferometer-Vorrichtung nach einem der Ansprüche 12 oder 13, **dadurch gekennzeichnet, dass** die Recheneinheit dazu ausgebildet ist, wenigstens vorbestimmte zweidimensionale Fourier-Koeffizienten der erfassten Kamerabilder zu errechnen.

15. Interferometer-Vorrichtung nach einem der Ansprüche 12 bis 14, **dadurch gekennzeichnet, dass** sie einen auf die Mittel (5, 6) zum Verändern der Referenzarmlänge wirkenden Stellantrieb und eine Stellantriebsteuerung aufweist, wobei die Stellantriebsteuerung mit der Recheneinheit zur Verarbeitung der erfassten Bilder kommunizierend verbunden ist.

16. Interferometer-Vorrichtung nach den Ansprüchen 14 und 15, **dadurch gekennzeichnet, dass** die Recheneinheit dazu ausgebildet ist, Fourier-Koeffizienten von erfassten Kamerabilder mit der Bilderfassung zeitlich Schritt haltend zu ermitteln und mit vorbestimmten Schwellenwerten zu vergleichen, und anhand des Vergleichsergebnisses vorbestimmte Befehle an die Stellantriebsteuerung zu senden.

**Claims**

1. A method for exposing a sectional face in the interior of a light scattering object (10), having the steps:

   providing a light source (1), which emits the light with a predetermined central wavelength and a coherence length of less than 25 microns;
   dividing the light from the light source (1) into sample light and reference light;
   laminary illuminating the object (10) with the sample light;
   feeding the sample light scattered from the object (10) to an electronic camera (8) with pixels of pixel width P;
   wherein the light scattered from the object (10) is fed to the electronic camera (8) such that the object (10) is imaged on the electronic camera (9), by setting up a mean speckle diameter D greater than two pixels wide along at least one axis in the camera (8) plane;
   causing reference light and sample light to interfere on the camera (8), by

   configuring a path length profile and a phase gradient of the reference light along the predetermined axis in the camera (8) plane, wherein the phase gradient has a contribution from the interval between $2\pi/D$ and $\pi/P$;
   acquiring of a camera image, wherein only the sample light scattered by a sectional face in the interior of the object (10) and having the path length profile of the reference light contributes to interference patterns.

2. The method according to claim 1, **characterized by** displacing the path length profile of the reference beam with a time-dependent velocity and acquisition of further camera images, each at least indexed with a measure of the time-dependent displacement of the path length profile.

3. The method according to claim 2, **characterized in that** the path length profile of the reference beam is displaced continuously, wherein all path lengths vary by no more than a predetermined fraction of the central wavelength during an exposure time of the camera (8).

4. The method according to any one of the preceding claims, **characterized by** separation of a camera image into a sectional image and a reflected image based on the phase gradient that is configured.

5. The method according to claim 4, wherein the separation of the camera image includes a two-dimensional Fourier filtering of the acquired camera image with respect to the phase gradient that is configured.

6. The method according to claim 4 or 5, **characterized in that** a three-dimensional model of the object (10) is calculated from a plurality of differently indexed camera images by the sectional image values being assigned to a voxel array, taking into account the relative displacement of images of identical structures in differently indexed reflected images.

7. The method according to any one of the preceding claims, **characterized in that** the light scattering object (10) is the retina of a living eye.

8. The method according to claim 7, **characterized in that** the sectional face is a plane intersecting the cell layers of the retina at an angle.

9. The method according to claim 2 and claim 8, **characterized in that**, from the relative displacement of the image of at least one layer boundary of the retina in differently indexed camera images, the change in the distance of the retina from the camera (8) between the acquisition times of the camera images is inferred.

10. The method according to any one of claims 7 to 9, **characterized by** displaying changes in the retina on the basis of the comparison of camera images and/or models with archived camera images and/or models of at least one previous acquisition time.

11. The method according to any one of claims 7 to 10, **characterized by** biometric verification of the identity of a user.

12. An interferometer device for the exposure of a volume of a scattering object (10) comprising:

    a light source (1), which emits light with a coherence length of less than 25 microns and central wavelength $\lambda 0$,
    a beam splitter (4) for dividing the light into a sample branch and reference branch,
    means (5, 6) for changing the reference branch length,
means (9) for surface illumination of the object (10) in the sample branch,
an electronic camera (8) (8) with pixels of width P,
a computing unit for processing the acquired camera images,
an optical imaging system (7, 9) with numerical aperture NA and magnification M arranged for feeding the light scattered by the object (10) to the camera (8), wherein the optical imaging system (7, 9) is configured to image the object (10) on the camera (8);
wherein the angle of incidence $\alpha$ of the light from the reference branch onto the camera (8) is configured in accordance with the condition $\lambda 0/(2*P) > \sin(\alpha) > NA/(1.22*M)$.

13. The interferometer device according to claim 12, **characterized in that** the imaging optical system (7, 9) comprises an eccentric aperture diaphragm.

14. The interferometer device according to either of claims 12 or 13, **characterized in that** the computing unit is configured to calculate at least predetermined two-dimensional Fourier coefficients of the acquired camera images.

15. The interferometer device according to any one of claims 12 to 14, **characterized in that** it has an actuator drive acting on the means (5, 6) for changing the reference branch length and an actuator controller, wherein the actuator controller is communicatively connected to the computing unit for processing the acquired images.

16. The interferometer device according to claims 14 and 15, **characterized in that** the computing unit is designed to identify Fourier coefficients of acquired camera images in step with the image acquisition and to compare them with predetermined threshold values, and to send predetermined commands to the actuator controller on the basis of the comparison result.

## Revendications

1. Procédé d'étanchéification d'une surface de coupe à l'intérieur d'un objet contrôlant la lumière (10) avec les étapes suivantes :

mise à disposition d'une source de lumière (1), qui émet une lumière avec une longueur d'onde centrale prédéterminée et une longueur de cohérence inférieure à 25 micromètres ;
répartition de la lumière de la source de lumière (1) en lumière d'échantillon et lumière de référence ; éclairage de la surface de l'objet (10) avec la lumière d'échantillon ;
introduction de la lumière d'échantillon diffusée par l'objet (10) dans une caméra électronique (8) avec des pixels de largeur P ;
la lumière d'échantillon diffusée par l'objet (10) étant introduite dans la caméra électronique de façon à ce que l'objet (10) soit représenté sur la caméra électronique (8), en générant un diamètre de taches moyen D supérieur à deux largeurs de pixels le long d'au moins un axe dans le plan de la caméra ; et
interférence de la lumière de référence et de la lumière d'échantillon sur la caméra (8), en générant un profil de longueur de trajet et un gradient de phase de la lumière de référence le long de l'axe prédéterminé dans le plan de la caméra, le gradient de phase présentant une valeur dans l'intervalle entre $2\pi/D$ et $\pi/P$ et
prise d'une image de caméra, seule la lumière d'échantillon diffusée par une surface de coupe à l'intérieur de l'objet (10) et présentant le profil de longueur de trajet de la lumière de référence contribuant aux motifs d'interférence.

2. Procédé selon la revendication 1, **caractérisé par** le déplacement du profil de longueur de trajet du rayon de référence avec une vitesse en fonction du temps et la prise d'autres images de caméra, chacune au moins indicée avec une mesure du déplacement en fonction du temps du profil de longueur de trajet.

3. Procédé selon la revendication 2, **caractérisé en ce que** le profil de longueur de trajet du rayon de référence est déplacé de manière continue, toutes les longueurs de trajets variant pendant un temps d'éclairage de la caméra (8) au maximum d'une fraction prédéterminée de la longueur d'onde centrale.

4. Procédé selon l'une des revendications précédentes, **caractérisé par** la séparation d'une image de caméra en une image de coupe et une image d'éclairage sur la base du gradient de phase généré.

5. Procédé selon la revendication 4, la séparation de l'image de caméra comprenant un filtrage de Fourier bidimensionnel de l'image de caméra saisie en ce qui concerne le gradient de phase généré.

6. Procédé selon la revendication 4 ou 5 et selon la revendication 2, **caractérisé en ce qu'**un modèle tridimensionnel de l'objet (10) est calculé à partir d'une pluralité d'images de caméra indicée différentes, en attribuant les valeurs d'image de coupe à une matrice de voxels en tenant compte du déplacement relatif des représentations de structures identiques dans différentes images incidentes indicées diffé-

**7.** Procédé selon l'une des revendications précédentes, **caractérisé en ce que** l'objet (10) diffusant la lumière est la rétine d'un oeil vivant.

**8.** Procédé selon la revendication 7, **caractérisé en ce que** la surface de coupe est un plan coupant les couches de cellules de la rétine avec un angle.

**9.** Procédé selon la revendication 2 et la revendication 8, **caractérisé en ce que**, à partir du déplacement relatif de la représentation d'au moins une limite de couche de la rétine, dans des images de caméra indicées différemment, on déduit la modification de la distance entre la rétine et la caméra (8) entre les moments de prise des images de la caméra.

**10.** Procédé selon l'une des revendications 7 à 9, **caractérisé par** la représentation de modifications de la rétine à l'aide de la comparaison d'images de caméra et/ou de modèles avec des images de caméra archivées et/ou des modèles d'au moins un moment de prise antérieur.

**11.** Procédé selon l'une des revendications 7 à 10, **caractérisé par** la vérification biométrique de l'identité d'un utilisateur.

**12.** Dispositif à interféromètre pour l'éclairage d'un volume d'un objet à contrôler, comprenant :

une source de lumière (1), qui émet une lumière avec une longueur de cohérence inférieure à 25 micromètres et une longueur d'onde centrale $\lambda o$,
un diviseur de faisceau (4) pour la répartition de la lumière dans un bras d'échantillon et un bras de référence,
des moyens (5, 6) pour la modification de la longueur du bras de référence,
des moyens (9) pour l'éclairage de la surface de l'objet (10) dans le bras d'échantillon,
une caméra électronique (8) avec des pixels de largeur P,
une unité de calcul pour le traitement des images de caméra saisies,
une optique de représentation (7, 9) avec une ouverture numérique NA et un agrandissement M, disposée pour l'introduction de la lumière diffusée par l'objet (10) dans la caméra (8), l'optique de représentation (7, 9) étant conçue de façon à ce qu'elle représente l'objet (10) sur la caméra (8) ;
l'angle d'incidence $\alpha$ de la lumière provenant du bras de référence sur la caméra (8) est conçu selon la condition $\lambda 0/(2*P) > \sin(\alpha) > NA/(1,22*M)$.

**13.** Dispositif à interféromètre selon la revendication 12, **caractérisé en ce que** l'optique de représentation (7, 9) comprend un diaphragme d'ouverture excentrique.

**14.** Dispositif à interféromètre selon l'une des revendications 12 ou 13, **caractérisé en ce que** l'unité de calcul est conçue pour calculer au moins des coefficients de Fourier bidimensionnels prédéterminés des images de caméra saisies.

**15.** Dispositif à interféromètre selon l'une des revendications 12 à 14, **caractérisé en ce qu'**il comprend un entraînement de réglage et un contrôle d'entraînement de réglage agissant sur les moyens (5, 6) de modification de la longueur du bras de référence, le contrôle d'entraînement de réglage étant relié en communication avec l'unité de calcul pour le traitement des images prises.

**16.** Dispositif à interféromètre selon les revendications 14 et 15, **caractérisé en ce que** l'unité de calcul est conçue pour déterminer des coefficients de Fourier d'images de caméra saisies en suivant le rythme de la prise d'images et pour les comparer avec des valeurs seuils prédéterminées et pour envoyer, à l'aide du résultat de la comparaison, des instructions prédéterminées au contrôle de l'entraînement de réglage.

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2008137933 A1
- US 2014092392 A1
- DE 4108944 A1

- US 2010020328 A1
- WO 2009111609 A2
- DE 102006015387 A1

**In der Beschreibung aufgeführte Nicht-Patentliteratur**

- **DRUCKSCHRIFT VON MASSATSCH et al.** Time-domain optical coherence tomography with digital holographic microscopy. *Applied Optics,* 01. April 2005, vol. 44